# FASCICULE DE BREVET EUROPEEN

(11) **EP 0 299 844 B1**
(45) Date de publication et mention de la délivrance du brevet: **26.05.1993**
(21) Numéro de dépôt: 88401734.4
(22) Date de dépôt: 04.07.1988
(51) Int. Cl.: G02C 7/16, G02F 1/13

(54) **Appareil pour la vue**
Sehapparat
Viewing apparatus

(30) Priorité: 07.07.1987 FR 8709642
(43) Date de publication de la demande: 18.01.1989
(73) Titulaire: COMMISSARIAT A L'ENERGIE ATOMIQUE, 75015 Paris Cédex 15 (FR); Klein, Siegfried, Dr., F-75009 Paris (FR)
(72) Inventeur: Klein, Siegfried, F-75009 Paris (FR)
(74) Mandataire: Mongrédien, André

(56) Documents cités:
- WO-A-81/02795
- BE-A- 563 709
- FR-A- 852 829
- FR-A- 1 265 417
- FR-A- 1 288 617
- FR-A- 2 151 623
- FR-A- 2 530 039
- GB-A- 1 131 284
- GB-A- 2 128 362
- GB-A- 2 170 613
- US-A- 4 012 129
- US-A- 4 462 661

## Description

La présente invention a pour objet un appareil pour la vue destiné à limiter l'intensité lumineuse instantanée traversant le cristallin de l'oeil sans diminution du champ visuel.

Cette invention trouve notamment une application dans le domaine médical pour les personnes atteintes de cataracte. Elle s'applique également dans tous les cas où une personne est susceptible d'être aveuglée par une intensité lumineuse trop importante. Elle peut donc être utilisée par exemple par un soudeur pour se protéger de l'intensité lumineuse émise par un plasma de soudure, ou par un automobiliste pour se protéger de l'intensité lumineuse émise par un phare de véhicule automobile.

Elle peut aussi être utilisée comme lunettes de soleil avec le grand avantage de ne pas avoir recours, pour la diminution de l'intensité lumineuse, à des verres teintés qui modifient considérablement la coloration de l'image réelle.

La cataracte est une maladie de l'oeil qui consiste en une décalcification entre les facettes du cristallin de l'oeil. Ceci produit une diffusion de la lumière qui traverse le cristallin, rendant ainsi la vision moins nette, c'est-à-dire voilée.

On sait traiter cette maladie par la chirurgie. Cependant, comme la maladie se développe lentement pendant plusieurs années, les personnes atteintes de cataracte supportent généralement leur maladie, avec une vision devenant de moins en moins nette donc de plus en plus voilée, pendant plusieurs années avant de recourir à une opération chirurgicale.

Les ophtalmologistes se servent parfois, pour évaluer le degré d'opacité du cristallin, d'un disque non transparent muni d'un petit orifice calibré. Le champ de vision de l'oeil est alors considérablement réduit, mais en même temps la fraction de l'image reçue par la rétine à travers cet orifice est infiniment plus nette.

L'amélioration de l'image perçue à travers ce petit orifice s'explique aisément. En effet, lorsque le cristallin est atteint de cataracte, comme déjà mentionné, la lumière est partiellement diffusée à l'intérieur de celui-ci avant d'atteindre la rétine. Pour un degré donné de décalcification l'importance de cette diffusion dépend essentiellement de deux facteurs qui sont d'une part l'intensité de la source lumineuse et d'autre part la fraction du cristallin traversé par ladite lumière. Autrement dit, lorsque la plaquette munie d'un petit orifice est placée devant l'oeil, la lumière est seulement diffusée dans une partie du cristallin, ce qui a évidemment comme conséquence que la diffusion totale à l'intérieur de celui-ci est fonction de la fraction du volume du cristallin éclairé. Par conséquent, la diffusion est réduite et l'image est plus nette.

On connaît également un dispositif destiné à atténuer l'intensité lumineuse, tel que décrit dans le document FR-A-1.288.617. Ce dispositif purement mécanique comporte deux disques animés d'un mouvement de rotation. Ces disques comprennent des secteurs alternativement transparents et opaques et sont placés devant les yeux.

L'invention a pour but un appareil destiné notamment à améliorer la vue d'une personne atteinte de cataracte, cet appareil ne comportant pas de pièce mécanique en mouvement.

De manière précise, l'invention a pour objet un appareil pour la vue, destiné à être porté devant les yeux d'une personne comprenant au moins une plaquette destinée à être portée devant les yeux d'une personne, caractérisée en ce que ladite plaquette est une cellule à cristaux liquides munie d'électrodes gravées reliées à des moyens électriques de balayage appliquant à ces électrodes des signaux électriques provoquant l'opacité de la totalité de la plaquette, à l'exception d'une bande de cette plaquette qui demeure transparente et qui balaye la plaquette à une fréquence fixée par les moyens électriques de balayage.

Le champ visuel est donc fractionné dans le temps. L'affaiblissement de l'intensité lumineuse est obtenu grâce à la fente se déplaçant par un balayage approprié. Comme au cinéma, lors du déplacement de cette bande, la persistance rétinienne intervient et de cette façon le champ visuel normal de l'oeil est transparent.

Les caractéristiques et avantages de l'invention ressortiront mieux de la description qui va suivre, donnée à titre illustratif et nullement limitatif, en référence aux dessins annexés, sur lesquels :
- la figure 1 illustre un mode de réalisation de l'invention dans lequel les plaquettes sont constituées de cellules à cristal liquide,
- la figure 2 illustre schématiquement un mode de réalisation du moyen de balayage de l'appareil de la figure 1,
- la figure 3a illustre schématiquement un mode de réalisation du moyen de chargement 22 du moyen de balayage, et
- la figure 3b illustre l'allure des signaux produits par ce moyen de chargement,
- la figure 4 représente un mode de réalisation de l'appareil de l'invention comportant une plaquette unique.

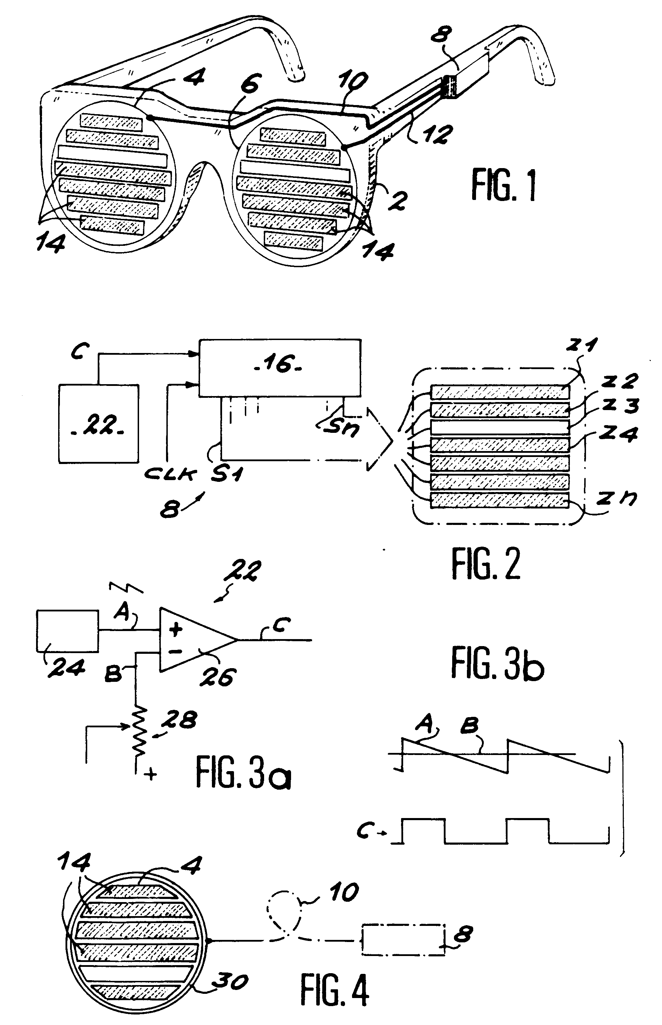

On a représenté sur la figure 1 un premier mode de réalisation de l'appareil de l'invention. Cet appareil comprend un support 2, de type monture de paire de lunettes, deux plaquettes 4, 6, et un moyen de balayage 8 muni de voies de commande 10, 12 reliées respectivement aux plaquettes 4, 6.

Chaque plaquette est constituée par une cellule à cristal liquide comprenant des électrodes gravées pour définir des bandes indépendantes. De manière classique, chaque bande peut être commandée électriquement de manière à être soit transparente, soit opaque.

Sur la figure 1, chaque plaquette a la forme d'un disque. Il est clair cependant que cette forme n'est pas nécessaire et que chaque plaquette peut par exemple être de forme carrée ou rectangulaire. De même, bien que les zones optiquement commandables soient représentées sous la forme de bandes horizontales, l'invention n'est pas limitée à ce mode de réalisation, mais peut au contraire être mise en oeuvre avec des zones actives de formes quelconques, telles que par exemple des secteurs circulaires.

Le moyen de balayage 8 peut être disposé sur le support 2 comme représenté sur la figure 1. Ce moyen de réalisation est avantageux si le moyen de balayage 8 est léger et peu volumineux. Dans le cas contraire, le moyen de balayage 8 peut être fixé sur un vêtement du malade ou mis dans une poche. Le moyen de balayage 8 émet des signaux électriques sur les voies 10 et 12 pour commander, de manière classique, l'état transparent ou opaque de chaque zone 14 de chaque plaquette 4, 6.

Les signaux électriques émis par le moyen de balayage 8 sont choisis de manière à ce qu'une zone transparente, constituée par une ou plusieurs bandes consécutives, balaie périodiquement la surface de la plaquette 4, par exemple de haut en bas. Un mode de réalisation de ce moyen de balayage 8 est représenté schématiquement sur la figure 2.

Ce moyen de balayage 8 comprend principalement un registre à décalage 16 comprenant une entrée série 18, et n sorties parallèles S₁, ..., Sₙ. Le moyen de balayage 8 comprend également un moyen de chargement 22 pour charger dans le registre à décalage 16 une suite de n bits. Le moyen de chargement 22 permet à l'utilisateur de fixer la largeur de la fente transparente. Ce moyen de chargement 22 délivre un signal C constitué par une suite de n bits, comprenant des bits de valeur "1" suivis de bits de valeur "0", le nombre de bits de valeur "1" déterminant la largeur de la fente transparente.

Chaque bit du registre à décalage 16 est associé à l'une des zones z₁, z₂, ..., zₙ d'une plaquette à cristal liquide. Les valeurs des bits émis sur les sorties S₁, ..., Sₙ du registre à décalage 16 déterminent l'état transparent ou opaque de la zone optique associée. Par exemple, un bit de valeur "1" engendre une tension électrique qui rend transparente la zone optique associée. Le balayage de la fente transparente constituée par une ou plusieurs zones consécutives est obtenu par un décalage cyclique des bits du registre à décalage 16 au moyen d'un signal d'horloge CLK. La fréquence de ce signal d'horloge est choisie en fonction du nombre de bandes n de manière que la fréquence de balayage de la plaquette soit de préférence supérieure à 16 Hz, ce qui, compte tenu de la persistance rétinienne, rend ce balayage invisible.

On a représenté schématiquement sur la figure 3a un mode de réalisation du moyen de chargement 22. Il comprend un générateur 24 pour délivrer un signal en dents de scie A, un amplificateur différentiel 26 comportant une entrée positive recevant le signal A, et une entrée négative, un potentiomètre 28, dont la valeur est ajustable par l'utilisateur, pour délivrer un signal B sur l'entrée inverseuse de l'amplificateur différentiel 26. Le signal C délivré par l'amplificateur différentiel 26 est appliqué sur l'entrée série 18 du registre à décalage 16 représenté sur la figure 2.

La figure 3B est un chronogramme illustrant l'allure des signaux A, B et C du moyen de chargement 22. L'amplitude du signal B est fixée par l'utilisateur au moyen du potentiomètre 28. Le signal C est périodique, de même période que le signal A délivré par le générateur 24. Ce signal C comporte, en début de chaque période, un créneau positif dont la largeur est fonction de l'amplitude du signal B. Cette largeur détermine le nombre de zones consécutives transparentes de chaque plaquette.

L'appareil selon l'invention présenté sur la figure 1 comporte deux plaquettes 4, 6 constituées chacune par une cellule à cristal liquide. Bien entendu, l'appareil de l'invention n'est pas limité au mode de réalisation de type "paire de lunettes", comportant deux plaquettes 4, 6, mais peut être appliqué également à un appareil pour la vue de type "monocle" comportant une seule plaquette.

Un tel appareil est représenté schématiquement sur la figure 4. Cet appareil comporte une plaquette 4 comportant une pluralité de bandes horizontales 14 dont l'état transparent ou opaque est commandable électriquement. Cette commande est réalisée par le moyen de balayage 8 à l'aide de signaux électriques transmis par une voie 10. Le support 30 de plaquette est ici constitué simplement par la circonférence de cette plaquette.

Dans chacun des modes de réalisation représentés sur les figures 1 et 4, les plaquettes ont une dimension de l'ordre de 35 mm et comportent 10 segments ayant chacun une largeur de 2 mm et séparés les uns des autres par des intervalles de l'ordre de 0,20 mm. La cellule utilise un cristal liquide de type nématique en hélice. Elle est normalement opaque, mais devient transparente sous l'effet d'une tension électrique.

Dans les modes de réalisation représentés sur les figures 1 et 4, les plaquettes sont constituées par des cellules à cristal liquide dans lesquelles le balayage de la fente est obtenu sans déplacement mécanique.

L'appareil de l'invention procure une amélioration sensible de la vision des personnes atteintes de cataracte grâce au faible champ de vision instantané. Le champ de vision total de l'oeil est balayé par déplacement de la fente à une fréquence de préférence supérieure à 16 Hz. Ce balayage n'est pas perçu à cause de la persistance rétinienne.

L'appareil de l'invention présente un intérêt pour les personnes atteintes de cataracte étant donné que ce procédé permet essentiellement de réduire considérablement la diffusion de la lumière traversant un cristallin atteint de cataracte et améliorer de ce fait la vision des personnes souffrant de cette maladie.

Il peut également être utilisé avantageusement pour les personnes qui sont susceptibles d'être soumises à une intensité lumineuse trop importante pour l'oeil. C'est le cas notamment des personnes qui réalisent des opérations de soudure et qui doivent se protéger de l'intensité lumineuse créée par le plasma de soudure. C'est également le cas des automobilistes qui peuvent être aveuglés, notamment la nuit, par les phares d'un véhicule automobile.

Il peut aussi être utilisé comme lunettes de soleil en présentant le grand avantage de diminuer l'intensité lumineuse, sans avoir recours à des verres teintés qui modifient considérablement la coloration de l'image réelle.

Dans tous les cas, l'appareil de l'invention permet de réduire l'intensité du rayonnement lumineux reçu sans diminuer le champ de vision.

## Revendications

1. Appareil pour la vue, destiné à être porté devant les yeux d'une personne, comprenant au moins une plaquette (4, 6) destinée à être portée devant les yeux d'une personne, caractérisé en ce que ladite plaquette est une cellule à cristaux liquides munie d'électrodes (14) gravées reliées à des moyens électriques de balayage (8) appliquant à ces électrodes des signaux électriques provoquant l'opacité de la totalité de la plaquette, à l'exception d'une bande de cette plaquette qui demeure transparente et qui balaye la plaquette à une fréquence fixée par les moyens électriques de balayage.

2. Appareil selon la revendication 1, caractérisé en ce que ladite fréquence de balayage est supérieure à 16 Hz.

## Claims

1. Viewing apparatus to be worn in front of the eyes of a person, comprising at least one plate (4, 6) to be worn in front of the eyes of a person, characterized in that said plate is a liquid crystal cell provided with etched electrodes (14) connected to electric scanning means (8) applying to said electrodes electric signals causing the opacity of the complete plate, with the exception of a strip thereof which remains transparent and which scans the plate at a frequency fixed by the electric scanning means.

2. Apparatus according to claim 1, characterized in that said scanning frequency exceeds 16 Hz.

## Patentansprüche

1. Sehapparat, dazu bestimmt, vor den Augen einer Person getragen zu werden, wenigstens eine kleine Platte (4, 6), die dazu bestimmt ist, vor den Augen einer Person getragen zu werden, enthaltend, dadurch gekennzeichnet, daß die Platte eine Flüssigkristallzelle ist, ausgestattet mit geätzten Elektroden (14), die mit elektrischen Einrichtungen zur Takterzeugung (8) verbunden sind, die auf diese Elektroden elektrische Signale wirken lassen, welche die Lichtundurchlässigkeit der gesamten Platte bewirken, mit Ausnahme eines Streifens dieser Platte, der transparent bleibt und der die Platte mit einer durch die elektrischen Einrichtungen zur Takterzeugung bestimmten Frequenz überstreicht.

2. Sehapparat nach Anspruch 1, dadurch gekennzeichnet, daß die Taktfrequenz über 16 Hz liegt.
